# EUROPÄISCHE PATENTSCHRIFT

(11) **EP 1 371 645 B1**
(45) Veröffentlichungstag und Bekanntmachung des Hinweises auf die Patenterteilung: **20.02.2013**
(21) Anmeldenummer: 03005232.8
(22) Anmeldetag: 11.11.1997
(51) Int. Cl.: C07D 207/12, C07D 211/40

(54) **Verwendung von (3S,2'R)-Glycopyrronium als Arzneimittel**
Use of (3S,2'R)-Glycopyrronium in medicaments
Utilisation de (3S,2'R)-glycopyrronium comme médicament

(30) Priorität: 11.11.1996 AT 197396
(43) Veröffentlichungstag der Anmeldung: 17.12.2003
(62) Teilanmeldung aus: 97911049.1
(73) Patentinhaber: MEDA Pharma GmbH & Co. KG, 61352 Bad Homburg (DE)
(72) Erfinder: Noe, Christian R., 1180 Wien (AT); Mutschler, Ernst, 55129 Mainz (DE); Lambrecht, Günter, 55129 Mainz (DE); Elgert, Michael, 60437 Frankfurt/Main (DE); Czeche, Sittah, 99867 Gotha (DE); Waelbroeck, Magali, 1070 Brüssel (BE)

(56) Entgegenhaltungen:
- FI-B- 49 713
- US-A- 4 721 722
- "CHEMICAL ABSTRACTS + INDEXES, AMERICAN CHEMICAL SOCIETY. COLUMBUS, US" CHEMICAL ABSTRACTS + INDEXES, AMERICAN CHEMICAL SOCIETY. COLUMBUS, US, Bd. 11, Nr. 119, 1993, Seite 82 XP002055749 ISSN: 0009-2258 & FUDER ET AL: "Glycopyrronium bromide blocks differentially responses mediated by muscarinic receptor subtypes" NAUNYN-SCHMIEDEBERG'S ARCH. PHARMACOL., Bd. 347, Nr. 6, 1993, Seiten 591-595,
- SLEEVI M C ET AL: "OPTICAL ISOMERS OF ROCASTINE AND CLOSE ANALOGUES: SYNTHESIS AND H1 ANTIHISTAMINIC ACTIVITY OF ITS ENANTIOMERS AND THEIR STRUCTURAL RELATIONSHIP TO THE CLASSICAL ANTIHISTAMINES" JOURNAL OF MEDICINAL CHEMISTRY, AMERICAN CHEMICAL SOCIETY. WASHINGTON, US, Bd. 34, Nr. 4, 1. April 1991 (1991-04-01), Seiten 1314-1328, XP002070895 ISSN: 0022-2623

## Beschreibung

Die vorliegende Erfindung betrifft die Verwendung pharmazeutisch geeigneter Salze von (3S,2'R)-Glycopyrronium als Arzneimittel.

Als pharmazeutisch geeignetes Salz wird das Salz einer pharmakologisch unbedenklichen Säure bezeichnet.

Ester von Aryl-cycloalkyl-hydroxysäuren mit cyclischen Alkoholen, in welchen ein quartärer Stickstoff vorhanden ist, und welche mit der allgemeinen Formel I 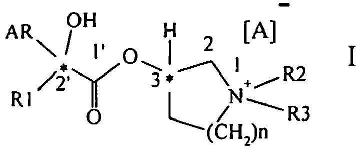 beschrieben werden, bestehend aus einer Hydroxycarbonsäure, in welcher AR einen aromatischen Ring bedeutet und in welcher R₁ einen cycloaliphatischen Ring bedeutet und bestehend aus einer Alkoholkomponente, in welcher sich die Hydroxylgruppe an einem Dimethylpyrrolidiniumring (n=1) oder Dimethylpiperidiniumring (n=2) befindet, in welchem R₂=R₃ ein Niederalkyl bedeutet, und in welcher A ein Halogenid bedeutet, wurden in einigen Fällen als Spasmolytika beschrieben. Wenn die beiden Reste R₂ und R₃ identisch sind, weisen Verbindungen der allgemeinen Formel I zwei Chiralitätszentren auf. Das eine Zentrum ist dem Säureteil zuzuordnen und betrifft die mit 2' bezeichnete Position, das zweite Chiralitätszentrum befindet sich im cyclischen Ringsystem an der mit 3 bezeichneten Position. Da Verbindungen dieser Struktur somit zwei Chiralitätszentren aufweisen, sind im Prinzip vier Stereoisomere (3R,2'R; 3S,2'R; 3R,2'S und 3S,2S') denkbar. Bisher wurden reine Stereoisomere der allgemeinen Formel I weder isoliert noch synthetisch hergestellt, oder - was für den Gegenstand der vorliegenden Erfindung grundlegend ist - pharmakologisch untersucht. Der wichtigste, auch in der Therapie eingesetzte Vertreter der allgemeinen Formel I ist Glycopyrroniumbromid (AR = Phenyl, R₁ = Cyclopentyl, R₂=R₃ = Methyl, n = 1, A = Br). Mit dem internationalen Freinamen Glycopyrroniumbromid wird das razemische Diastereoisomerengemisch, in welchem alle vier Stereoisomere enthalten sind, beschrieben.

Bisher bekanntgewordene Publikationen und Patente befassen sich entweder mit dem als Stereoisomerengemisch vorliegenden Wirkstoff Glycopyrroniumbromid (CAS 596-51-0), mit erythro-(RN 59677-73-5) bzw. threo- (RN 59677-70-2) konfigurierten Racematen des tert. Aminoesters (CRN 131118-11-1 ), welche lediglich als Vorstufe bei der Synthese der Verbindungen der Formel I angesehen werden können, oder dem Stereoisomerengemisch des analogen Cyclohexyl-Derivates (R₁ = Cyclohexyl) der allgemeinen Formel I (mit n = 1) (RN 101564-29-8). In den Publikationen J. Chem. Soc. Perkin Trans. 2, 1973, (14), 1875-9, und J. Chem. Soc. Perkin Trans. 2, 1973, (14), 1963-6, sind Ergebnisse von Kristallstrukturanalysen der als Stereoisomerengemische vorliegenden Verbindungen Glycopyrroniumbromid und Hexapyrroniumbromid beschrieben. Die Publikationen Oyo Yakuri, 1973, 7(6), 881-7; Oyo Yakuri, 1973, 7(6), 889-901, und Acta Anaesthesiol. Scand., 1978, 22(4), 447-57, beschreiben Ergebnisse von pharmakologischen Untersuchungen mit dem Stereoisomerengemisch der Verbindung Glycopyrroniumbromid bzw. von Kombinationspräparaten dieser Substanz mit Neostigmin und Pyridostygmin. Die Publikationen Finn. Chem. Lett.., 1975, (3-4), 94-6, und FI 49713 beschreiben die teilweise Trennung des Stereoisomerengemisches auf dem Wege einer Kristallisation mit 5-Nitroisophthalsäure und die NMR-Untersuchung der bereits oben erwähnten threo- bzw. erythro-konfigurierten Racemate. Hierbei gelang den Autoren, ausgehend vom Stereoisomerengemisch (CRN 131118-11-1), lediglich die Diastereomerentrennung in die zwei Razemate, jedoch keine Herstellung der enantiomerenreinen Verbindungen. Die Publikationen Fresenius Z. Anal. Chem., 1981, 308(5), 413-27; Science, 1986, 1132-1135; J. Liq. Chromatogr., 1990, Bd. 13, Nr.4, S. 779-787; Acta Anaesthesiol. Scand., 1978, 22(4), 447-57, sowie das europäische Patent EP 128886 A2 beschreiben Ergebnisse von Studien zur chromatographischen Analytik des Stereoisomerengemisches der Verbindung Glycopyrroniumbromid bzw. die Herstellung der verwendeten stationären Phasen. In keiner der aufgeführten Publikationen wird eine Enantiomerentrennung bzw. Isolierung der einzelnen Stereoisomere der allgemeinen Formel I berichtet. Eine HPL-chromatographische Trennung gelang in allen aufgeführten Fällen nur auf der Stufe der Diastereomeren. H. Fuder, M. Meincke, Naunyn-Schmiedeberg's Arch. Pharmacol. 1993, 347(6), 591-5, beschreiben die Blockierung von Muskarinrezeptoren durch Glycopyrroniumbromid in Experimenten mit Organpräparaten. Das verwendete Glycopyrroniumbromid war in allen beschriebenen Versuchen eine Mischung verschiedener Stereoisomerer; einzelne enantiomerenreine Verbindungen standen nicht zur Verfügung. Die Herstellung der enantiomerenreinen Verbindung gemäß dieser Erfindung ist aus dem Stand der Technik noch nicht bekannt.

Die pharmakologische Wirkung von Arzneistoffen der allgemeinen Formel I basiert auf ihrer Interaktion mit muskarinischen Acetylcholinrezeptoren (Muskarinrezeptoren). Sie werden daher als m-Cholinozeptor-Antagonisten, bzw. Parasympatholytika oder - wegen ihrer erschlaffenden Wirkung auf die glatte Muskulatur - als neurotrope Spasmolytika bezeichnet. Die vielfältigen Wirkungen der Parasympatholytika umfassen: Beschleunigung der Herzfrequenz. Reduktion der Sekretion der Tränen-, Speichel- und Schweiß-Sekretion sowie der Drüsen des Verdauungstraktes, Erschlaffung der glatten Muskulatur der Bronchien, des Magen-Darm-Kanals, der Gallenwege, Uteren und der Harnblase, Erweiterung der Pupillen und Akkomodationsstörung. Quartäre Spasmolytika, zu welchen auch die Verbindungen gemäß der allgemeinen Formel I gehören, überwinden die Blut-Hirn-Schranke nicht und sind daher zentral unwirksam. Je nach Art der Anwendung sind die gewünschten und unerwünschten Wirkungen von Parasympatholytika verschieden. Verwendet man diese Substanzen als Spasmolytika, so wird man beispielsweise die verminderte Speichelsekretion oder die Pupillenerweiterung als Nebenwirkung bezeichnen.

Aufgrund der Forschungen der letzten Jahre ist es bekannt, daß Muskarinrezeptoren keine einheitliche Struktur besitzen, sondern daß die pharmakologischen Wirkungen auf Interaktionen mit mindestens vier verschiedenen Muskarinrezeptor-Subtypen zurückzuführen sind. Diese weisen einerseits eine unterschiedliche Verteilung in verschiedenen Organen auf, andererseits sind bei manchen neuronalen Signalübertragungskaskaden verschiedene Muskarinrezeptor-Subtypen mit verschiedenen Funktionen involviert. Verschiedene Wirkungen bzw. Nebenwirkungen lassen sich auf Interaktionen mit den verschiedenen Rezeptorsubtypen zurückführen, so daß eine hohe Subtypspezifität ein Ziel bei der Entwicklung moderner Spasmolytika ist. Glycopyrroniumbromid ist ein lange etablierter Wirkstoff, der den Anforderungen eines "modernen" Therapeutikums dieses Typs nicht entspricht. Glycopyrroniumbromid ist jedoch nicht nur ein Razemat, sondern darüber hinaus ein Diastereomerengemisch, bei welchem je nach Herstellungsprozedur die Verhältnisse der einzelnen Isomere im Produkt sogar schwanken können. Somit kann es bei solchen isomeren Wirkstoffgemischen zu zufälligen Subtypprofilen kommen, wodurch ein gezielter Einsatz erschwert und das Auftreten von unerwünschten Nebenwirkungen provoziert wird.

Häufig liegt bei der Trennung des Razemates eines Arzneistoffes in Enantiomere die pharmakologische Wirkung ausschließlich bei einem der Enantiomere. Aus Beispiel 4, vor allem aus der logarithmisch aufgetragenen Graphik Fig. 1, kann entnommen werden, daß bei Glycopyrronium alle Isomere im Prinzip Rezeptoraffinität aufweisen können. Jedoch zeigen die einzelnen Enantiomere zum einen deutliche Unterschiede in ihren Affinitäten, zum anderen ergeben sich auch deutliche Abweichungen in der Subtypspezifität M₁ - M₄, wobei die Unterschiede in den Affinitäten maximal einen Faktor von etwa 1000 ausmachen. Gerade die hohe Affinität zum M₃-Rezeptor-Subtyp bei einer relativ niederen Affinität zum M₂-Rezeptor-Subtyp macht die bevorzugten höher affinen Enantiomere (etwa im Beispiel 4: 1b und 1c) zu besonders geeigneten Wirkstoffen zur Therapie von Spasmen der glatten Muskulatur des Magen-Darm-Traktes und des Urogenitaltraktes sowie zur Behandlung obstruktiver Atemwegserkrankungen. Durch ein besonders günstiges Subtypprofil und eine durch ihre hohe Affinität bedingte besonders niedrige Dosierungsmöglichkeit bringen sie einen effizienteren therapeutischen Erfolg bei deutlich reduziertem Nebenwirkungspotential.

Ein weiterer besonders wichtiger Faktor bei der therapeutischen Anwendung von Enantiomeren der allgemeinen Formel I besteht in der kinetischen Subtypselektivität. Wie Beispiel 5 bzw. Fig. 2 entnommen werden kann, liegen die Dissoziationshalbwertszeiten der einzelnen Enantiomere 1a - 1d der Verbindungen der allgemeinen Formel I (AR = Phenyl, R₁ = Cyclopentyl, R₂= R₃= Methyl, n = 1, A = I) beim M₃-Rezeptorsubtyp zwischen 1 Minute und 120 Minuten, während die Dissoziationshalbwertzeiten an den Subtypen M₁, M₂ und M₄ im Bereich von wenigen Minuten liegen. Gerade Verbindungen mit einer besonders langen Dissoziationshalbwertszeit erlauben wegen ihrer starken Haftung eine besonders niedere Dosierung bei lange anhaltendem therapeutischem Effekt. Die Möglichkeit, durch gezielte Auswahl eines Enantiomers mit bestimmter Dissoziationshalbwertszeit die Dauer der pharmakologischen Wirkung gezielt zu beeinflussen, stellt einen weiteren wichtigen Fortschritt der gegenständlichen Verbindungen gegenüber dem Stand der Technik dar. Die beschriebenen Eigenschaften waren nicht vorhersehbar, auch gab es keine Hinweise in der Literatur.

Zusammenfassend läßt sich feststellen, daß sich enantiomerenreine Ester der allgemeinen Formel I gegenüber dem Stand der Technik durch ihre pharmakodynamische Selektivität auszeichnen. Sie besitzen in der bevorzugt beanspruchten Konfiguration eine deutlich höhere Affinität zu muskarinischen M₃- als zu M₂-Rezeptoren und zeigen darüber hinaus eine kinetische Selektivität für M₃-Rezeptoren, d.h. sie diffundieren nur langsam von diesem Rezeptortyp. Aufgrund dieser Eigenschaften eignen sie sich ganz besonders zur Therapie von Spasmen der glatten Muskulatur des Magen-Darm-Kanals und des Urogenitattraktes sowie zur Behandlung obstruktiver Atemwegserkfankungen, wie Asthma bronchiale und chronische Bronchitis. Im Vergleich zu den bisher angewandten, nichtselektiven Parasympatholytika weisen sie aufgrund ihrer definierten Subtypselektivität deutliche Unterschiede in den pharmakologischen Eigenschaften auf. Im Vergleich zu bekannten Stereoisomerengemischen oder Razematen können die Verbindungen zudem in besonders niedriger Dosierung eingesetzt werden (Vermeidung von enantiomerem Ballast!). Aus diesen Gründen sind Nebenwirkungen mit Sicherheit in deutlich geringerem Umfang zu erwarten.

Demgemäß ist die Verwendung eines pharmazeutisch geeigneten salzes on enantiomerenreinem [(Cyclopentylhydroxyphenylacetyl)oxy]-1,1-dimethyl-pyrrolidinium zur Herstellung eines Arzneimittels zur Therapie von Spasmen der glatten Muskulatur des Magen-Darm-Kanals sowie zur Behandlung obstruktiver Atemwegserkrankungen (Asthma bronchiale, chronische Bronchitis) Gegenstand der Erfindung.

Besonders bevorzugt beschrieben ist die Verwendung von (3S,2'R)-3-[(Cyclopentylhydroxyphenylacetyl)oxy]-1,1-dimethyl-pyrrolidinium mit hoher M3-**Subtypselektivität** (pKi grösser als 10) und großen Dissoziationshalbwertszeiten am M3-Rezeptor in Arzneimitteln zur Behandlung obstruktiver Atemwegserkrankungen, bevorzugt Asthma bronchiale und chronische Bronchitis.

Die beschriebene Erfindung wird nunmehr durch die folgenden Beispiele erläutert. Verschiedenartige, andere Ausgestattungen werden für den Fachmann aus der vorliegenden Beschreibung ersichtlich. Es wird jedoch ausdrücklich darauf hingewiesen, dass die Beispiele und die Beschreibung lediglich zur Erläuterung vorgesehen und nicht als Einschränkung der Erfindung anzusehen sind.

### Beispiele:

### Beispiel 1 (Referenzbeispiel):

### Herstellung von (3S,2'S) 3-[(cyclopentylhydroxyphenylacetyl)oxy]-1,1-dimethyl-pyrrolidinium-iodid 1 a (allg. Formel I, Ar = Phenyl, R1 = Cyclopentyl, R2 = R3 = Methyl, n=1, A = I)

In einer trockenen Reaktionsapparatur werden 20 mmol (3S)-1-Methyl-3-pyrrolidinol und 24 mmol 2-Cyclopentyl-2-hydroxy-phenylessigsäuremethylester in 500 ml n-Heptan abs. vorgelegt. Anschliessend werden 200 ml Heptan zum Entfernen aller Feuchtigkeitsspuren überdestilliert und durch den Wasserabscheider abgelassen. Nach dem Abkühlen werden 2 mmol NaH oder NaOMe (10 mol%) zugesetzt und wiederum zum Sieden erhitzt. Die Temperatur wird so gewählt, dass das n-Heptan nur langsam überdestilliert. Die übergehende Menge wird über 5-6 h fortlaufend aus dem Tropftrichter ersetzt, bis der Hydroxyester vollständig umgesetzt ist. Nach wässriger Aufarbeitung des Reaktionsgemisches und Extraktion mit Ether wird das Rohprodukt über Na₂SO₄ / K₂CO₃ 2:1 getrocknet. Nach Absaugen vom Trockenmittel wird im Eisbad vorgekühlt und unter Eiskühlung bis zur Sättigung mit etherischer HCI / 2-Butanon versetzt. Hierbei fällt das Produkt zunächst ölig an. Durch Zusatz von 2-Butanon bzw. durch Abdestillieren von Ether erhält man eine klare Lösung, aus der unter Eiskühlung das Hydrochlorid des Diastereomerengemisches (3S,2'R/S)-3-(2-cyclopentylhydroxy-phenylacetoxy)-1-methyl-pyrrolidin (allg. Formel IV Ar = Phenyl, R₁ = Cyclopentyl, R₂ = Methyl, n = 1) auskristallisiert. Ausbeute 15,7 mmol. Fp. 176°C.

### Herstellung der Hydrogentartrate: Diastereomerentrennung durch fraktionierende Kristallisation:

Zur Herstellung der Hydrogentartrate überführt man 15 mmol des oben beschriebenen Hydrochlorids mit NaHCO₃ / K₂CO₃ Puffer pH 10 in einen Scheidetrichter und extrahiert die wäßrige Phase dreimal mit je 150 ml Diethylether. Die vereinten etherischen Phasen werden mit 100 ml Ethylacetat versetzt und über Na₂SO₄ / K₂CO₃ 2:1 getrocknet . Nach Absaugen vom Trockenmittel wird am Rotationsverdampfer auf ca. 100 ml Volumen reduziert. Die Lösung wird auf ca. 60°C erhitzt und mit einer Lösung von 1,2 eq (18mmol) enantiomerenreiner Weinsäure in Ethylacetat versetzt. Das Hydrogentartrat kristallisiert über Nacht im Kühlschrank. Durch mehrfaches Umkristallisieren lassen sich die Diastereomeren bis zu einem de von 99% trennen. Ausbeute 8,3 mmol. (3S,2'S) 3-[(cyclopentylhydroxyphenylacetyl)oxy]-1-methyl-pyrrolidin, D-(-)-Hydrogentartrat allg. Formel IV (AR = Phenyl, R₁ = Cyclopentyl, R₂ = Methyl, n = 1): Smp.178-179°C ¹H-NMR-Spektrum (300 MHz CD₄O): δ (ppm) 1,3-1,7 (M,8H,Cyclopentyl-CH₂), 2,05-2,1 (M,1H,C4), 2,39-2,46 (M,1H,C4), 2,77 (S,3H,N-Methyl), 2,97-3,0 (M,1H,Cyclopentyl C1), 3,18-3,25 (dd,1H,C2, ²J =12,8 Hz,³J=0-1 Hz), 3,31-3,5 (M,2H,C5), 3,6-3,7 (dd,1H,3J=5,2Hz,2J=13Hz,C2), 4,42 (S,2H,Tartrat), 5,34-5,39 (M,1H,C3), 7,2-7,8 (M,5H) Zuordnung aufgrund H,H-COSY-NMR

### Quarternierung:

Nach Freisetzen der Basen durch Extraktion mit Ether gegen Bicarbonat-Puffer pH 10 und Trocknen über Na₂SO₄ / K₂CO₃ 2:1 wird durch Zugabe von 3 eq (20 mmol) Methyliodid quarterniert und das kristallin anfallende Produkt (3S,2'S)-3-[(cyclopentylhydroxyphenylacetyl)oxy]-1,1-dimethyl-pyrrolidinium-iodid 1a (allg. Formel I, AR = Phenyl, R₁ = Cyclopentyl, R₂ = R₃ = Methyl, n = 1, A = I) abgesaugt. (Die Bestimmung der Diastereomerenüberschüsse kann durch HPLC-Methoden an β-Cyclodextrin- und "Whelck"-Phasen bzw. durch Auswertung von NMR-Spektren der oben beschriebenen Hydrogentartrate erfolgen.)

(3S,2'S)- 3-[(cyclopentylhydroxyphenylacetyl)oxy]-1,1-dimethyl-pyrrolidinium-iodid (1a) allg. Formel I (AR = Phenyl, R₁ = Cyclopentyl, R₂ = R₃ = Methyl, n = 1, A = I) Smp. 165°C ¹H-NMR (300MHz CD₄O) δ (ppm) (1,15-1,4 (M,2H), 1,5-1,7 (M,6H),Cyclopentyl-Methylen), 2,2 (M,1H,C 4), 2,63 (M,1H,C 4), 2,9 (S,3H,N-Methyl), 2,93-2,99 (M,1H,Cyclopentyl-Methin), 3,1 (S,3H,N-Methyl), 3,43-3,47 (dd,1H,C2,²J=14Hz,³J=0Hz), 3,5-3,7 (M,2H,C5), 3,75 (dd,1H,C2,²J=13,7Hz,³J=6,05Hz), 5,38 (M,1H,C3), 7,15-7,4 (M,3H), 7,5-7,65 (M,2H) ¹³C-NMR 52 MHz CD₄O δ (ppm) (24,4-25,4) (4t,Cyclopentyl-methylen), 28,5 (t,C4), 47,4 (t,Cyclopentyl-Methin), 51,3 (q,N-Methyl), 51,8 (q,N-Methyl), 63,6 (t,C5), 68,9 (t,C2), 72,0 (d,C3), 78,4 (s,Hydroxyester C2), 124,5 (d), 126,1 (d), 126,7 (d), 140,5 (s), 172,4 (s)

Die Bestimmung der Diastereomerenreinheit (de) erfolgte durch Vergleich der Integrale der N-Methyl-protonen der diastereomeren Hydrogentartrate in ¹H-NMR-Spektren (300 MHz, CD₄O), bzw. durch HPLC-Analytik an β-Cyclodextrinphasen (Cyclobond β-CD-OH, 50*0,4 cm, Puffer: 85%H₂O, 15% CH3CN, 0,2% CH3COOH V/V, 0,35 ml/min isokratisch, UV-Detektion: 236 nm).

### Beispiel 2:

Herstellung von (3S,2'R) 3-[(cyclopentylhydroxyphenylacetyl)oxy]-1,1-dimethyl-pyrrolidinium-iodid 1b (allg. Formel 1, Ar = Phenyl, R₁ = Cyclopentyl, R₂ = R₃ = Methyl, n = 1, A = 1)

Aus den Mutterlaugen der unter Beispiel 1 beschriebenen Diastereomerentrennung wird durch Zusatz von Ether das D-(-)-Hydrogentartrat der (3S,2'R)-konfigurierten Verbindung kristallisiert. Mehrfaches Umkristallisieren führt zu einem de von > 98%.
(3S,2'R)- 3-[(cyclopentylhydroxyphenylacetyl)oxy]-1-methyl-pyrrolidin, D-(-)-Hydrogentartrat (allg. Formel IV AR = Phenyl, R₁ = Cyclopentyl, R₂ = Methyl, n = 1): Smp.158-160°C ¹H-NMR (300 MHz CD₄O): δ (ppm) 1,3-1,7 (M,8H,Cyclopentyl-CH₂), 2,0-2,1 (M,1H,C4), 2,39-2,46 (M,1H,C4), 2,81 (S,3H,N-Methyl), 2,93-3,05 (M,1H,Cyclopentyl C1), 3,24-3,4 (M,3H,C2,C5), 3,63-3,7 (dd,1H, ³J=5,2Hz, ²J=13Hz, C2), 4,42 (S,2H,Tartrat),5,38 (M,1H,C3), 7,2-7,7 (M,5H) Zuordnung aufgrund H,H-COSY-NMR.

Die Quarternierung erfolgte ebenfalls wie oben beschrieben und liefert das kristallin anfallende Produkt (3S,2'R) 3-[(cyclopentylhydroxyphenylacetyl)oxy]-1,1-dimethyl-pyrrolidinium-iodid 1b (allg. Formel I, AR = Phenyl, R₁ = Cyclopentyl, R₂ = R₃ = Methyl, n = 1, A = I). Smp.108-109°C ¹H-NMR (300MHz CD₄O): δ (ppm) (1,15-1,4 (M,2H), 1,5-1,7 (M,6H),Cyclopentyl-Methylen), 2,2 (M,1H,C 4), 2,65-2,85 (M,1H,C 4), 3,01 (M,1H,Cyclopentyl-Methin), 3,06 (S,3H,N-Methyl), 3,1 (S,3H,N-Methyl), 3,55-3,8 (M,3H,C2,C5), 4,07 (dd,1H,C2,²J=13,8Hz,³J=6,2Hz), 5,48 (M,1H,C3), 7,26-7,4 (M,3H), 7,5-7,65 (M,2H).
¹³C-NMR (50 MHz CD₄O) / DEPT und CH-Korelation): δ (ppm) (27,0 (t), 27,4 (t), 27,41 (t), 28,06 (t),Cyclopentyl-methylen), 31,26 (t,C4), 46,6 (t,Cyclopentyl-Methin), 53,8 (q,N-Methyl), 54,3 (q,N-Methyl), 66,2 (t,C5), 71,5 (t,C2), 74,5 (d,C3), 81,2 (s,Hydroxyester C2), 127 (d), 128,8 (d), 129,3 5 (d), 143,2 (s), 175,0 (s).

Die Bestimmung der Diastereomerenreinheit erfolgte wie in Beispiel 1 beschrieben.

### Beispiel 3 (Referenzbeispiel):

Herstellung von (3R,2'R)- 3-[(cyclopentylhydroxyphenylacetyl)oxy]-1,1-dimethyl-pyrrolidinium-iodid 1c (allg. Formel I, Ar = Phenyl, R₁ = Cyclopentyl, R₂ = R₃ = Methyl, n = 1, A = I)

Die Herstellung erfolgte wie unter Beispiel 1 beschrieben, ausgehend von (3R)-1-Methyl-3-pyrrolidinol unter Verwendung von L(+)-Weinsäure zur Diastereomerentrennung. Das unter Beispiel 1 beschriebene Verfahren liefert (3R,2'R)- 3-[(cyclopentylhydroxyphenylacetyl)oxy]-1,1-dimethyl-pyrrolidinium-iodid 1c (allg. Formel I, Ar = Phenyl, R₁ = Cyclopentyl, R₂ = R₃ = Methyl, n = 1, A = I). Die [¹H]- und [¹³C]-NMR analytischen Daten stimmen mit jenen der in Beispiel 1 aufgeführten (3S,2'S)-konfigurierten Verbindung 1a überein. Smp. 165-166°C

Die Bestimmung der Diastereomerenreinheit erfolgte wie in Beispiel 1 beschrieben.

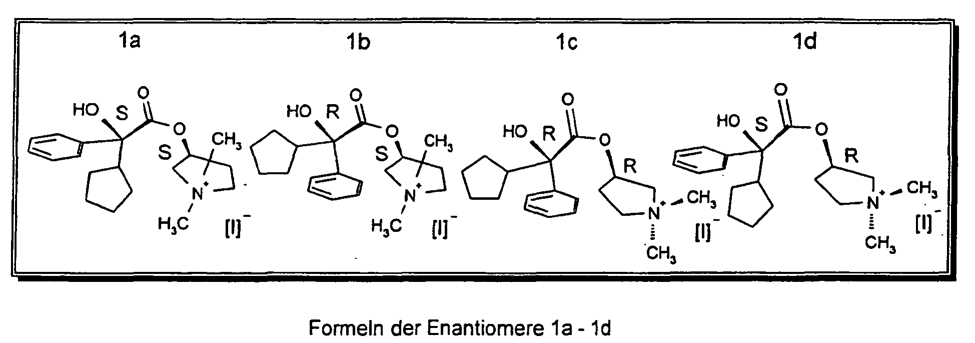

### Beispiel 4:

Pharmakologische Daten von Verbindungen der allg. Strukturformel I mit
AR=Phenyl, R₁ = Cyclopentyl, n= 1, R₂ = R₃ = Methyl, A = lodid

**Tab.1**

| pharmakologische Affinitätsdaten der Verbindungen la-d | | | | | | | |
|---|---|---|---|---|---|---|---|
| Verbindung Abs.Konf.^{§1} | pA₂ | pKᵢ | pA₂^{a} | pKᵢ^{c} | pEC₅₀^{a} | PKᵢ^{b} | pKᵢ^{b} |
| | RVD (M1) | M1 | GPA (M2) | M2 | GPI (M3) | M3 | M4 |
| | | | | | | | |
| (3S,2'S) Ia | 8,22 | 8,36 | 7,92 | 7,88 | 6,82 | 7,82 | 7,82 |
| (35,2'R) Ib | 10,40 | 10,48 | 9,39 | 9,74 | 9,39 | 10,50 | 10,30 |
| (3R,2'R) Ic | 10,30 | 10,18 | 9,43 | 9,63 | 8,76 | 10,2 | 10,27 |
| (3R,2'S) Id | 9,53 | 9,36 | 8,69 | 9,00 | 8,57 | 9,63 | 9,40 |

| | | | | | | | |
|---|---|---|---|---|---|---|---|
| a pA₂ und pEC₅₀ Werte aus funktionellen Experimenten am Kaninchen-Vas-Deferens, Meerschweinchen-Atrium und Meerschweinchen-Ileum b pKᵢ-Werte aus [³H]-NMS-Bindungsstudien an M₁-, M₃-,M₄-Humanrezeptoren aus CHO-K1-Zellen c pKᵢ-Werte aus [³H]-NMS-Bindungsstudien an M₂-Rezeptoren aus Rattenherzpräparationen d pA₂ = 7,61 | | | | | | | |

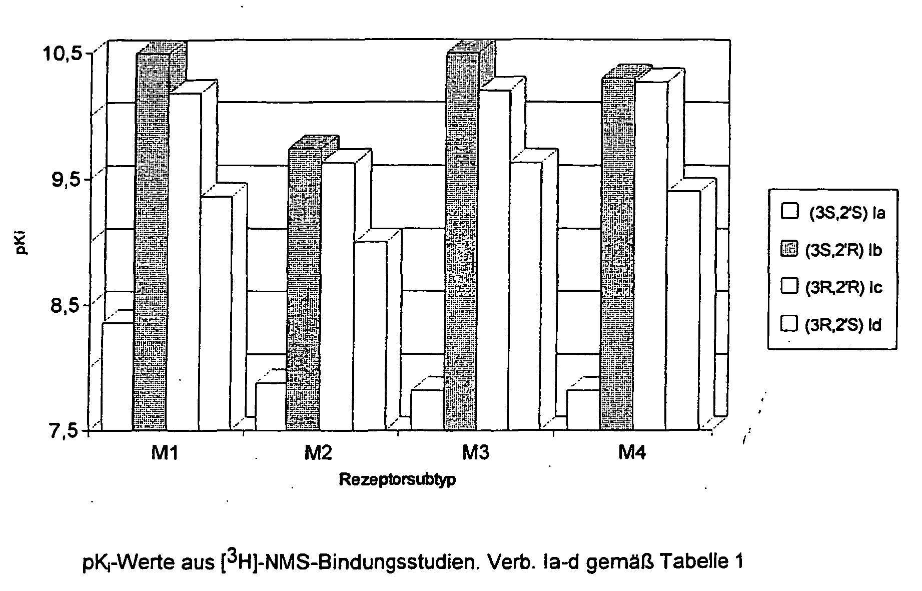

### Beispiel 5:

Kinetik-Daten am M3-Rezeptorsubtyp von Verbindungen der allg. Strukturformel I mit
AR=Phenyl, R₁ = Cyclopentyl, R₂ = R₃ = Methyl, n= 1, A = lodid

**Tab.2**

| Assoziations- und Dissoziationskonstanten, Dissoziations-halbwertszeiten am M3-Rezeptorsubtyp | | | |
|---|---|---|---|
| Verb.ME-X¹ | k on^{e} | k off^{f} | t_{½}[min]^{g} |
| (3S,2'S) Ia | 0,052 | 0,8000 | 1 |
| (35,2'R) Ib | 0,410 | 0,0100 | 70 |
| (3R,2'R)Ic | 0,160 | 0,0060 | 120 |
| (3R,2'S) * Id | 0,028 | 0,0080 | 90 |
| **e** Assoziationskonstante nmol/min, | | | |
| **f** Dissoziationskonstante nmol/min, | | | |
| **g** Dissoziationshalbwertszeit in Minuten. | | | |
| Kinetik-Daten aus NMS-Bindungsstudien an M3-Rezeptorsubtypen aus CHO-K1-Zellinien | | | |

| | | | |
|---|---|---|---|
| 1 Die Zuordnung der Absolutkonfiguration in 2-Position der Hydroxysäuren erfolgte durch Vergleich der CD-Spektren mit den entsprechenden Cyclohexyl-Mandelsäuren. Die Zuordnung der Absolutkonfiguration der 2-Cyclohexyl-2-hydroxy-phenylessigsäuren erfolgte an Hand der Drehwerte gemäß T.D. Inch et. al. J. Chem. Soc. 1968 S.1693-1699. | | | |
| * analytische Untersuchungen zeigten im nachhinein, daß das damals verwendete (3R,2'S)-Enantiomer mit ca 20% Anteil des hochwirksamen (3R,2'R)-Enantiomers verunreinigt war. Die Halbwertszeit für das reine (3R,2'S)-Enantiomer liegt wahrscheinlich nur bei ca 1-5 min | | | |

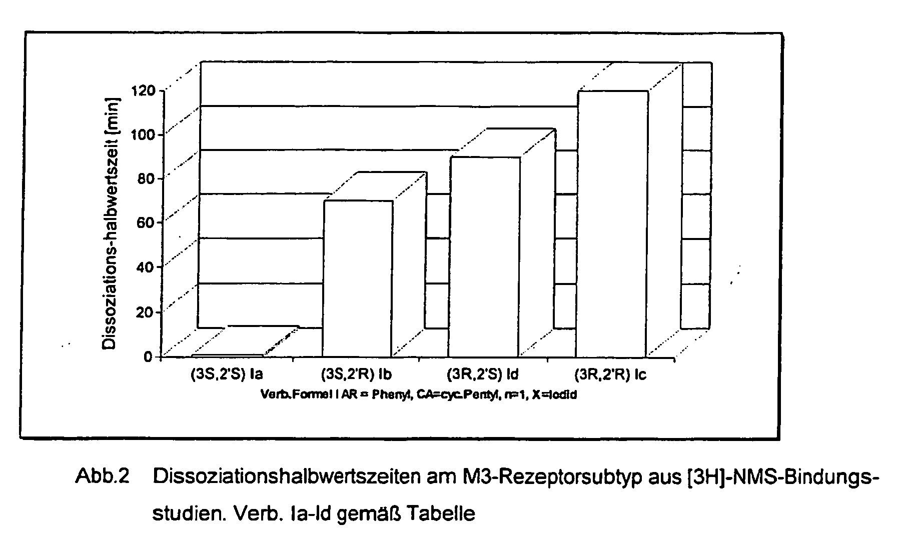

### Beispiel 6 (Referenzbeispiel):

(3*R*,2'*R*)-3-[(2'-Cyclopentyl-2'-hydroxy-2'-phenylacetyl)oxy]-1,1-dimethylpyrrolidiniumbromid (allg. Formel I, Ar = Phenyl, R1 = Cyclopentyl, R2 = R3 = Methyl, n = 1, A⁻ = Br⁻)

In einer trockenen Reaktionsapparatur werden 5 mmol (3R)-1-Methyl-3-pyrrolidinol und 5 mmol (2*R*)-2-Cyclopentyl-2-hydroxy-2-phenylessigsäuremethylester in 150 ml n-Heptan abs. vorgelegt. Anschließend werden 100 ml Heptan zum Entfernen aller Feuchtigkeitsspuren destillativ entfernt und durch den Wasserabscheider abgelassen. Nach dem Abkühlen werden 0,5 mmol NaOMe (10 mol%) zugesetzt und wiederum zum Sieden erhitzt. Die übergehende Menge Lösungsmittel wird über 5-6 h fortlaufend mittels Tropftrichter ersetzt. Nach wäßriger Aufarbeitung des Reaktionsgemisches und Extraktion mit Ether wird die organische Phase über Na₂SO₄ / K₂CO₃ 2:1 getrocknet. Entfernen von Trocken- und Lösungsmittel liefert die freie Base.

¹H-NMR (Benzol-d₆) (300 MHz): δ = 7.94 (d, ³J=7.33Hz, 2H, Phenyl 2"und 6"-CH); 7.36-7.15 (m, 3H, Phenyl, 3", 5"und 4"-CH, Benzol [7.25]); 5.15-5.08 (m, 1H, 3-CH); 4.24 (s, 1H, -OH); 3.16-3.03 (m, 1H, Cyclopentyl-Methin); 2.62-2.47 (m, 2H, 2-CHH und 5-CHH); 2.24 (dd, ²J=10,87Hz, ³J=5,9Hz, 1H, 2-CHH (3'*R*,2*R*)); 2.09 (s, 3H, NCH₃, (3'*R*,2*R*)); 2.04-1.91 (m, 2H, 5-CHH 4-CHH); 1.89-1.52 (m, 6H, 4-CHH, Cyclopentyl-Methylen); 1.51-1.35 (m, 3H, Cyclopentyl-Methylen) ¹³C-NMR (Benzol-d₆) (50 MHz): δ = 175.6 (s, 1-COO); 142.8 (s, Phenyl-1"C); 128.3 (d, Phenyl, 3"und 5"-C); 127.6 (d, Phenyl, 4"-C); 126.4 (d, Phenyl 2"und 6"-C); 79.6 (s, 2-C); 76.9 (d, 3'-C); 61.6 (t, 2'-C); 54.8 (t, 5'-C); 47.8 (d, Cyclopentyl-Methin); 41.6 (q, NCH₃); 32.8 (t, 4'-C); 27.3/26.9/26.8/26.5 (t, Cyclopentyl-Methylen)

### Quarternierung:

Die freie Base wird durch Zugabe von 3 eq Methylbromid, gelöst in tert-Butylmethylether, quarterniert und das kristallin anfallende Produkt abgesaugt.

Die [¹H]- und [¹³C]-NMR analytischen Daten des quartären Salzes stimmen mit jenen der in Beispiel 1 aufgeführten (33,2'S)-konfigurierten Verbindung 1a überein.

Die Bestimmung der Absolutkonfiguration erfolgte mittels Röntgenstrukturanalyse.

### Beispiel 7:

(3*S*,2'*R*)-3-[(2'-Cyclopentyl-2'-hydroxy-2'-phenylacetyl)oxy]-1,1-dimethylpyrrolidiniumbromid (allg. Formel I, Ar = Phenyl, R1 = Cyclopentyl, R2 = R3 = Methyl, n = 1, A⁻ = Br⁻)

Die Herstellung erfolgt nach der unter Beispiel 6 angegebenen allgemeinen Vorschrift aus (3S)-Methylpyrrolidinol und (2R)- 2-Cyclopentyl-2-hydroxy-2-phenyl)essigsäuremethylester. NMR-Daten der freien Base:
¹H-NMR (Benzol-d₆) (300 MHz): δ = 7.94 (d, ³J=7.33Hz, 2H, Phenyl 2"und 6"-CH); 7.36-7.15 (m, 3H, Phenyl, 3", 5"und 4"-CH, Benzol [7.25]); 5.15-5.08 (m, 1H, 3-CH); 4.24 (s, 1H, -OH); 3.16-3.03 (m, 1H, Cyclopentyl-Methin); 2.62-2.47 (m, 2H, 2-CHH und 5-CHH); 2.40 (dd, ²J=10.83Hz, ³J=5.85Hz, 2-CHH); 2.13 (s, NCH₃); 2.04-1.91 (m, 2H, 5-CHH 4-CHH); 1.89-1.52 (m, 6H, 4-CHH, Cyclopentyl-Methylen); 1.51-1.35 (m, 3H, Cyclopentyl-Methylen)

Die [¹H]- und [¹³C]-NMR analytischen Daten des quartären Salzes stimmen mit jenen der in Beispiel 2 aufgeführten (3S,2'R)-konfigurierten Verbindung 1b überein.

## Patentansprüche

1. Verwendung eines pharmazeutisch geeigneten Salzes von (3S,2'R)-3-[(Cyclopentylhydroxyphenylacetyl)oxy]-1,1-dimethyl-pyrrolidinium zur Herstellung eines Arzneimittels zur Behandlung von Spasmen der glatten Muskulatur des Magen-Darm-Kanals sowie zur Behandlung obstruktiver Atemwegserkrankungen.

## Claims

1. Use of a pharmaceutically suitable salt of (35,2'R)-3-[(cyclopentylhydroxyphenylacetyl)oxy]-1,1-dimethylpyrrolidinium for preparing a medicament for the treatment of spasms of the smooth muscles of the gastrointestinal tract and for the treatment of obstructive disorders of the respiratory tract.

## Revendications

1. Utilisation d'un sel pharmaceutiquement approprié de (35,2'R)-3-[cyclopentylhydroxyphényl-acétyl)oxy]-1,1-diméthyl-pyrrolidinium pour la fabrication d'un médicament destiné au traitement de spasmes des muscles lisses du tractus gastro-intestinal ainsi qu'au traitement de maladies obstructives des voies respiratoires.
